(19) Europäisches Patentamt
European Patent Office
Office européen des brevets

(11) Veröffentlichungsnummer: **0 012 801**
**B1**

(12) # EUROPÄISCHE PATENTSCHRIFT

(45) Veröffentlichungstag der Patentschrift: **07.04.82**

(51) Int. Cl.³: **C 07 C 87/455**, A 61 K 31/135

(21) Anmeldenummer: **79103906.8**

(22) Anmeldetag: **11.10.79**

(54) **2-(1-Phenyl-2,5-cyclohexadienyl)-äthylaminderivate, Verfahren zu deren Herstellung, deren Verwendung als pharmazeutische Wirkstoffe sowie diese enthaltende Arzneimittel.**

(30) Priorität: **11.10.78 CH 10552/78**

(43) Veröffentlichungstag der Anmeldung: **09.07.80 (Patentblatt 80/14)**

(45) Bekanntmachung des Hinweises auf die Patenterteilung: **07.04.82 Patentblatt 82/14**

(84) Benannte Vertragsstaaten: **AT BE CH DE FR GB IT LU NL SE**

(56) Entgegenhaltungen:
**DE - A - 1 518 545**
**DE - A - 1 518 663**
**DE - A - 1 907 909**
**DE - A1 - 2 619 617**
**DE - B - 1 284 421**
**DE - B - 1 298 527**
**FR - M - 7 031**
**J. chem. Soc. 1951, S. 2524**
**J. org. Chem., Vol. 19, (1954), S. 615**

(73) Patentinhaber: **F. HOFFMANN-LA ROCHE & CO. Aktiengesellschaft**

**CH-4002 Basel (CH)**

(72) Erfinder: **Müller, Peter M., Dr.**
**Gempenweg 32 A**
**CH-4144 Arlesheim (CH)**
Erfinder: **Pfister, Rudolf, Dr.**
**Neubadstrasse 128**
**CH-4054 Basel (CH)**
Erfinder: **Urban, René, Dr.**
**Emanuel Büchelstrasse 2**
**CH-4052 Basel (CH)**

(74) Vertreter: **Lederer, Franz, Dr. et al**
**Patentanwälte Dr. Franz Lederer Reiner F. Meyer**
**Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 012 801 B1**

Imprimerie Jouve, 18, rue St-Denis, 75001 Paris, France

# 2-(1-Phenyl-2,5-cyclohexadienyl)-äthylaminderivate, Verfahren zu deren Herstellung, deren Verwendung als pharmazeutische Wirkstoffe sowie diese enthaltende Arzneimittel

Die vorliegende Erfindung betrifft Cyclohexadien-Derivate und zwar solche der allgemeinen Formel

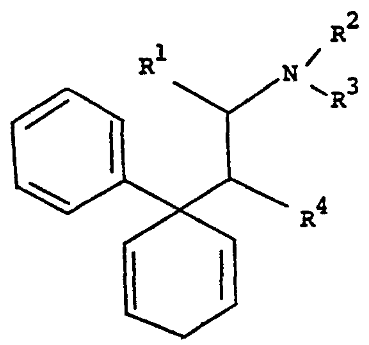

(I)

worin $R^1$ Wasserstoff oder Methyl, $R^2$ Wasserstoff, Methyl oder Aethyl, $R^3$ Methyl oder Aethyl und $R^4$ Wasserstoff oder Methyl bedeuten, wobei von den Resten $R^1$ und $R^4$ mindestens einer Wasserstoff bedeutet (einschliesslich der optisch aktiven Antipoden solcher Verbindungen, die ein asymmetrisches Kohlenstoffatom aufweisen), sowie Säureadditionssalze davon.

Gegenstand der vorliegenden Erfindung sind die neuen Cyclohexadien-Derivate der allgemeinen Formel I, einschliesslich deren optische Antipoden, Säureadditionssalze davon, die Herstellung dieser Verbindungen, ferner pharmazeutische Präparate enthaltend diese Verbindungen und die Herstellung solcher Präparate sowie die Verwendung dieser Verbindungen bzw. von pharmazeutischen Präparaten enthaltend diese Verbindungen bei der Bekämpfung von Schmerzen.

In der Deutschen Auslegeschrift No. 1.518.545 wird die Herstellung von basisch substituierten Phenylcyclohexan-Derivaten beschrieben, welche coronargefässerweiternde Wirkungen besitzen. Die Französische Patentschrift No. 7.031 M betrifft Arzneimittel, welche basisch substituierte Phenylcyclohexan-Derivate enthalten und spasmolytische — und teilweise auch analgetische — Eigenschaften besitzen. Im Gegensatz dazu werden in der vorliegenden Anmeldung basisch substituierte Phenylcyclohexadien-Derivate beschrieben, welche ausgeprägte und weitgehend selektive analgetische Eigenschaften besitzen.

Die Cyclohexadien-Derivate der allgmeinen Formel I und deren Säureadditionssalze können erfindungsgemäss dadurch hergestellt werden dass man

a) Biphenyl in Gegenwart von Lithium oder Calcium und Ammoniak mit einer Verbindung der allgemeinen Formel

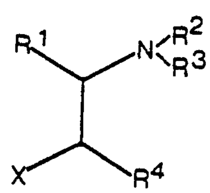

(II)

(gegebenenfalls in Form eines optischen Antipoden und/oder Säureadditionssalzes) worin $R^1$, $R^2$, $R^3$ und $R^4$ die genannte Bedeutung haben und X für eine Abgangsgruppe steht,
umsetzt, oder

b) eine Verbindung der allgemeinen Formel

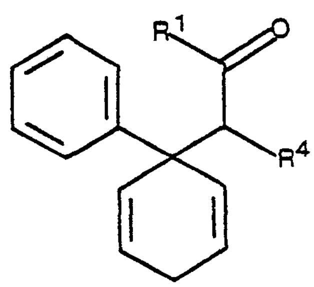

(III)

worin $R^1$ und $R^4$ die genannte Bedeutung haben, unter reduzierenden Bedingungen mit einem Amin der allgemeinen Formel

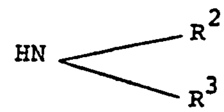

(IV)

worin $R^2$ und $R^3$ die genannte Bedeutung haben, umsetzt oder

c) eine Verbindung der allgemeinen Formel

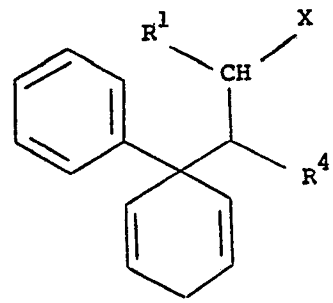

(V)

worin $R^1$ und $R^4$ die genannte Bedeutung haben und X für eine Abgangsgruppe steht, mit einem Amin der Formel IV umsetzt, oder

d) eine Verbindung der allgemeinen Formel

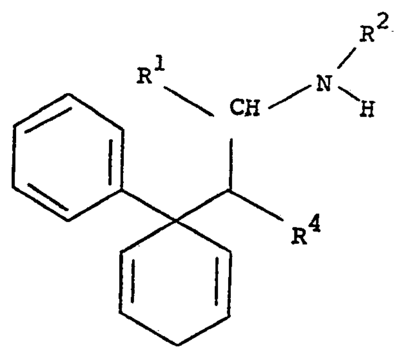

(VI)

worin $R^1$, $R^2$ und $R^4$ die genannte Bedeutung haben, entsprechend alkyliert, oder

e) eine Verbindung der allgemeinen Formel

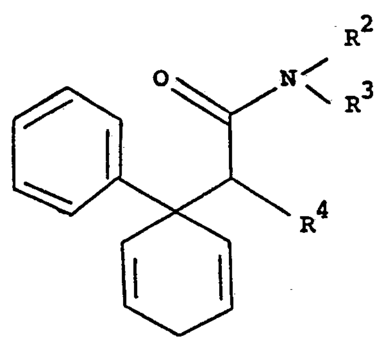

(VII)

worin $R^2$, $R^3$ und $R^4$ die genannte Bedeutung haben, reduziert, worauf man gewünschtenfalls ein erhaltenes Racemat in die optisch aktiven Antipoden aufspaltet und ein erhaltenes Produkt gewünschtenfalls in ein Säureadditionssalz überführt.

Die Umsetzung nach der Variante a) des erfindungsgemässen Verfahrens erfolgt zweckmässig in einem Lösungsmittel, wie Aether, Tetrahydrofuran, Monoglym, Diglym oder Dioxan, und bei einer Temperatur zwischen −80° und −30 °C. Als Abgangsgruppe X kommt z.B. Brom, Jod, Mesyl oder Tosyl, insbesondere Chlor, in Betracht.

Die Umsetzung nach Variante b) des erfindungsgemässen Verfahrens erfolgt vorzugsweise in Gegenwart eines Lösungsmittels, z.B. einem niederen Alkanol, wie Methanol, Aethanol und Isopropanol, und bei einer Temperatur zwischen −10° und 50 °C. Als Reduktionsmittel kommen Borhydride, wie Na-Cyanborhydrid oder Natriumborhydrid in Betracht.

Die Umsetzung nach der Variante c) des erfindungsgemässen Verfahrens erfolgt vorzugsweise in einem inerten organischen Lösungsmittel, wie z.B. Toluol, Benzol, Xylol, Monoglym oder Diglym, und bei einer Temperatur zwischen 120 und 180 °C. Vorzugsweise wird die Umsetzung unter Druck, z.B. 10-40 atü durchgeführt. Als Abgangsgruppe X kommt von den oben genannten insbesondere Mesyl in Betracht.

Die N-Alkylierung nach der Variante d) des erfindungsgemässen Verfahrens kann z.B. mit einem Alkylierungsmittel, wie einem Alkylhalogenid oder einem Dialkylsulfat, durchgeführt werden. Anderseits kann die Alkylierung auch reduktiv erfolgen, z.B. mit Formaldehyd und Ameisensäure oder durch Acylierung und Reduktion, z.B. mit Lithiumaluminiumhydrid.

Für die Herstellung von Verbindungen der allgemeinen Formel I mit $R^1$ = Wasserstoff nach der Variante e) des erfindungsgemässen Verfahrens kommen als Reduktionsmittel sehr reaktive komplexe Hydride, wie Lithiumaluminiumhydrid und DIBAH, in Betracht. Die Reduktion erfolgt vorzugsweise in Gegenwart eines inerten organischen Lösungsmittels, wie Aether, Tetrahydrofuran, Monoglym, Diglym oder Toluol, sowie bei einer Temperatur zwischen 0 und 100 °C.

Die als Ausgangsmaterialien verwendeten Verbindungen der allgemeinen Formel II sind — soweit sie racemisch oder nicht chiral sind — bekannt oder können in Analogie zur Herstellung der bekannten Vertreter hergestellt werden.

Salze, z.B. die Hydrochloride, von optisch aktiven Verbindungen der Formel II können z.B. erhalten werden, wenn man optisch aktive Alkohole der allgemeinen Formel

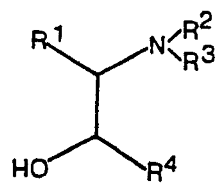

(VIII)

worin $R^1$-$R^4$ die genannte Bedeutung haben,
in Form eines Salzes, z.B. als Hydrochlorid, in Methylenchlorid mit Thionylchlorid umsetzt.

Die optisch aktiven Alkohole der Formel VIII sind bekannt oder können in Analogie zur Herstellung der bekannten Vertreter hergestellt werden.

Die Ausgangsverbindungen der allgemeinen Formel III mit $R^1$ = H können durch Oxydation einer Verbindung der allgemeinen Formel

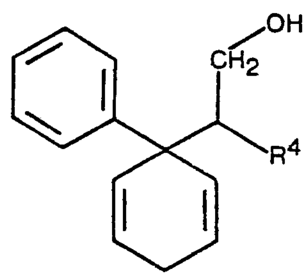

(IX)

worin $R^4$ die genannte Bedeutung hat,
erhalten werden. Die Oxydation kann mittels Schwefeltrioxydpyridinkomplex in Dimethylsulfoxid (DMSO) erfolgen.

Die Ausgangsverbindungen der allgemeinen Formel V mit $R^1$ = H können erhalten werden, indem man einen Ester der Formel

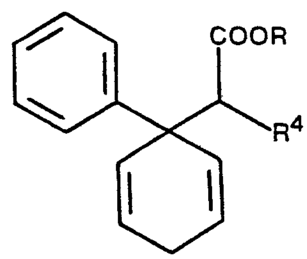

(X)

worin $R^4$ dasselbe wie oben und R niederes Alkyl bedeutet,
zu einem Alkohol der Formel IX reduziert und diesen verestert.

Die Verbindungen der Formel X können erhalten werden, indem man Biphenyl in Gegenwart von Lithium oder Calcium und ammoniak mit einem Chloressigsäureester der allgemeinen Formel

$$Cl - \overset{R^4}{\overset{|}{CH}} - COOR \qquad (XI)$$

worin R nieders Alkyl bedeutet und $R^4$ die genannte Bedeutung hat, umsetzt.

Ausgangsverbindungen der Formel III mit $R^1$ = Methyl können aus den Ausgangsverbindungen der Formel III mit $R^1$ = H z.B. dadurch erhalten werden, dass man letztere mit Methylmagnesiumjodid oder -bromid behandelt und die entstandenen Verbindungen der Formel

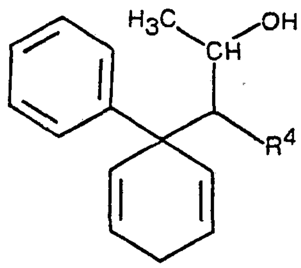

(XII)

worin $R^4$ dasselbe wie oben bedeutet, mit Schwefeltrioxydpyridinkomplex in DMSO zur Ausgangsverbindung der Formel III mit $R^1$ = Methyl aufoxydiert. Alkohole der allgemeinen Formel XII können zu den Ausgangsverbindungen der allgemeinen formel V mit $R^1$ = Methyl verestert werden.

Die Ausgangsamine der allgemeinen Formel IV sind bekannt.

Die Ausgangsamine der allgemeinen Formel VI mit $R^1 = R^2 = H$ können dadurch erhalten werden, dass man Biphenyl in Gegenwart von Lithium oder Calcium und Ammoniak mit einer Verbindung der allgemeinen Formel

$$Cl - \overset{R^4}{\overset{|}{CH}} - CN \qquad (XIII)$$

umsetzt und das erhaltene Nitril der allgemeinen Formel

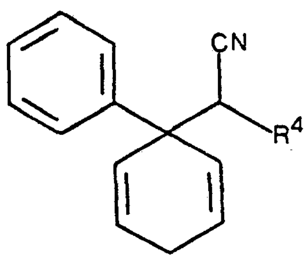

(XIV)

worin $R^4$ dasselbe wie oben bedeutet, mit z.B. Lithiumaluminiumhydrid reduziert.

Die Amine der Formel VI mit $R^2$ = H können auch dadurch hergestellt werden, dass man eine Verbindung der Formel V mit Ammoniak umsetzt. Die Amine der Formel VI mit $R^2$ = Methyl oder Aethyl kann man durch Umsetzen einer Verbindung der Formel V mit Monomethyl- bzw. Monoäthylamin erhalten.

Die Ausgangsamide der allgemeinen Formel VII können z.B. erhalten werden, indem man Biphenyl in Gegenwart von Lithium oder Calcium und Ammoniak mit einem Amid der allgemeinen Formel

$$Cl - \overset{R^4}{\overset{|}{CH}} - CO - NR^2R^3 \qquad (XV)$$

worin $R^2$, $R^3$ und $R^4$ die genannte Bedeutung haben, umsetzt.

In Form von Racematen anfallende Endprodukte der Formel I können nach an sich bekannten Methoden, beispielsweise durch Racematspaltung mit optisch aktiven Säuren, in die Antipoden aufgetrennt werden.

Verbindungen der Formel I können durch Behandlung mit anorganischen oder organischen Säuren in entsprechende Salze übergeführt werden, wobei insbesondere pharmazeutisch verträgliche Salze von Bedeutung sind. Beispiele von Säuren, welche pharmazeutisch verträgliche Salze bilden, sind Chlorwasserstoffsäure, Bromwasserstoffsäure, Salpetersäure, Schwefelsäure, Phosphorsäure, Weinsäure, Zitronensäure, Maleinsäure, Ascorbinsäure, Ameisensäure, Essigsäure, Bernsteinsäure, Methan-, Benzol- und p-Toluolsulfonsäure, usw.

Die erfindungsgemässen Verbindungen sind — wie im bekannten « Writhing-Test » resp. « Kaolin-Test » nachgewiesen wurde — analgetisch wirksam. Ihre Wirkungsstärke ist etwas geringer als diejenige von Codein und Propoxyphen jedoch grösser als diejenige von Aminophenazon und Acetylsalicylsäure. Im Vergleich zu Codein und Propoxyphen zeichnen sich die erfindungsgemässen Verbindungen jedoch durch geringere unerwünschte Nebenwirkungen aus, insbesondere — durch geringere bzw. fehlende Suchterzeugung. Die erfindungsgemässen Verbindungen können demnach bei der Bekämpfung von Schmerzen verwendet werden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Einzeldosis von 100-300 mg und eine Tagesdosis von 400-1 200 mg angemessen sein.

Von besonderem Interesse sind die Verbindungen der Formel I mit

a) $R^1 = R^4 = H$ und $R^2 = R^3 = CH_3$(N,N-Dimethyl-1-phenyl-2,5-cyclohexadien-1-äthylamin).,

b) $R^1 = R^4 = H$ und $R^2 = R^3 = C_2H_5$(N,N-Diäthyl-1-phenyl-2,5-cyclohexadien-1-äthylamin),

c) $R^1 = CH_3$, $R^4 = H$, und $R^2 = R^3 = CH_3$($\alpha$-N,N-Trimethyl-1-phenyl-2,5-cyclohexadien-1-äthylamin),

wobei die sub a) genannte Verbindung ganz besonderes Interesse beansprucht.

Die erfindungsgemässen Verbindungen können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, lokal oder perkutan, z.B. in Form von Salben, Crèmes, Gelées, Lösungen, oder parenteral, z.B. in Form von Injektionslösungen, erfolgen.

Zur Herstellung von Tabletten, Lacktabletten, Dragées und Hartgelatinekapseln können die erfindungsgemässen Verbindungen mit pharmazeutisch inerten, anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc. ; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z.B. Wasser, Polyole, Saccharose, Invertzucker, Glukose und dergleichen.

Für Injektionslösungen eignen sich als Excipientien z.B. Wasser, Alkohole, Polyole, Glycerin, vegetabile Oele etc.

Für Suppositorien, lokale oder perkutane Anwendungsformen eignen sich als Excipientien z.B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugmittel oder Antioxidantien enthalten. Sie können auch noch andere therapeutische wertvolle Stoffe enthalten.

In den nachfolgenden Beispielen sind die Temperaturen in °Celsius angegeben.

## Beispiel 1

15,4 g (0,1 Mol) Biphenyl werden in 300 ml absolutem Aether bei −33° zu 600 ml kondensiertem, trockenem Ammoniak unter Rühren zugegeben. Dann werden 1,53 g (0,22 Mol) Lithium-Draht (in ca. 2 cm langen Stücken) ; entfettet mit Cyclohexan) innerhalb von 15 Minuten eingetragen, und es wird 1 Stunde nachgerührt. Hierauf wird eine Lösung von 23,7 g (0,22 Mol) 2-Chlor-N,N-dimethyläthylamin in 100 ml absolutem Aether rasch zugetropft. Es wird 20 Minuten nachgerührt und anschliessend wie folgt aufgearbeitet :

Es werden 11,8 g (0,22 Mol) festes Ammoniumchlorid eingetragen, worauf der Ammoniak völlig abdestilliert wird. Hierauf werden 200 ml destilliertes Wasser zugegeben und die Phasen werden getrennt. Die wässrige Phase wird mit Natriumhydroxyd auf pH > 12 gestellt und zweimal mit 200 ml Aether extrahiert. Die organischen Phasen werden vereinigt und zweimal mit 200 ml 2 N wässriger Salzsäure ausgezogen. Die salzsauren Auszüge werden wieder auf pH > 12 gestellt und zweimal mit 200 ml Aether extrahiert. Die Aetherauszüge werden mit Natriumchlorid und mit Natriumsulfat getrocknet, filtriert und eingedampft.

Es resultiert ein Oel, welches nach zweimaliger Kugelrohrdestillation (89-91°/0,02 Torr) N,N-Dimethyl-1-phenyl-2,5-cyclohexadien-1-äthylamin liefert.

Dieses Produkt wird mit Maleinsäure wie üblich (unter Verwendung von Aceton als Lösungsmittel) ins Maleat übergeführt und aus Aceton/Aether umkristallisiert. Das N,N-Dimethyl-1-phenyl-2,5-cyclohexadien-1-äthylamin-maleat (1: 1) wird 3 Stunden bei Raumtemperatur am Hochvakuum getrocknet und zeigt einen Schmelzpunkt von 130-132°.

## Beispiel 2

Zu 300 ml kondensiertem, trockenem Ammoniak werden 23,1 g (150 mMol) Biphenyl in 200 ml Aether

gegeben, worauf anschliessend bei −40° 2,1 g (300 mMol) Lithiumdraht (ca. 2 cm lange Stücke, entfettet mit Cyclohexan) im Verlauf von 15 Minuten zugegeben werden. Dann wird das Reaktionsgemisch während 2 Stunden bei Siedetemperatur (ca. −33°) gehalten. Anschliessend werden bei ca. −70° 7,2 g (50 mMol) 2-Chlor-N,N-dimethyläthylamin-hydrochlorid portionenweise rasch zugegeben. Das Reaktionsgemisch wird wieder auf Siedetemperatur erwärmt und während 1/2 Stunde bei dieser Temperatur gehalten. Nach anschliessender Zugabe von Ammoniumchlorid, Destillation des Ammoniaks und wässriger Aufarbeitung erhält man ein öliges Produkt. Nach Reinigung durch Chromatographie an der 20-fachen Menge Silicagel (Eluiermittel Methylenchlorid/Methanol : 10/1) wird das erhaltene N,N-Dimethyl-1-phenyl-2,5-cyclohexadien-1-äthylamin wie in Beispiel 1 beschrieben in das (1: 1)-Maleat übergeführt.

Beispiel 3

Zu 600 ml trockenem, kondensiertem Ammoniak werden bei ca. −33° unter Rühren 46,1 g (0,3 Mol) Biphenyl in 400 ml absolutem Aether zugegeben. Dann wird bei ca. −50° 4,2 g (0,6 Mol) Lithiumdraht (ca. 2 cm lange Stücke, entfettet mit Cyclohexan) innert 15 Minuten zugegeben. Das Reaktionsgemisch wird während 2 Stunden auf Siedetemperatur gehalten. Dann wird auf ca. −70° gekühlt, worauf 15,8 g (0,1 Mol) racemisches 2-Chlor-N,N-1-trimethyläthylamin-hydrochlorid portionenweise im Verlauf von 10 Minuten zugegeben werden. Es wird wieder auf Siedetemperatur (ca. −33°) erwärmt und bei dieser Temperatur während 2 Stunden gerührt. Dann werden 32 g (0,6 Mol) Ammoniumchlorid zugegeben und der Ammoniak wird abdestilliert. Anschliessend wird wässrig aufgearbeitet : Die Aether-Lösung wird mit Wasser gewaschen. Die wässrige Phase wird mit Natriumhydroxydlösung auf pH > 12 gestellt und noch zweimal mit Aether extrahiert. Die Aetherphasen werden vereinigt und zweimal mit 2 N wässriger Salzsäure extrahiert. Die sauren Auszüge werden wieder alkalisch gestellt und mit Aether extrahiert. Die Aetherphasen werden getrocknet, filtriert und eingeengt. Man erhält ein öliges Produkt, welches an der 20-fachen Menge Silicagel unter Verwendung von Methylenchlorid/Methanol (10 : 1) als Lösungsmittel α,N,N-Trimethyl-1-phenyl-2,5-cyclohexadien-1-äthylamin liefert. Dieses Produkt wird in Aether gelöst und mit Chlorwasserstoff ins Hydrochlorid übergeführt, welches aus Methylenchlorid/Aether umkristallisiert wird. Das Produkt zeigt den Schmelzpunkt 158-159°.

Beispiel 4

Zu 600 ml trockenem, kondensiertem Ammoniak werden bei ca. −33° unter Rühren 15,4 g (0,1 Mol) Biphenyl in 300 ml absolutem Aether zugegeben. Dann werden 1,72 g (0,25 Mol) Lithiumdraht (ca. 2 cm lange Stücke ; entfettet mit Cyclohexan) eingetragen. Es wird 1 Stunde nachgerührt. Dann wird eine Lösung von 24,3 g (0,2 Mol) 1-Dimethylamino-2-chlorpropan in 100 ml absolutem Aether zugegeben. Es wird 20 Minuten nachgerührt, worauf wie in Beispiel 1 beschrieben aufgearbeitet wird. Chromatographie des die Amine enthaltenden Auszugs an 500 g Aluminiumoxyd (Aktivität II) mit Methylenchlorid liefert β,N,N-Trimethyl-1-phenyl-2,5-cyclohexadien-1-äthylamin, welches mit äthanolischer Salzsäure ins Hydrochlorid übergeführt und aus Isopropanol umkristallisiert wird. Das Produkt zeigt den Schmelzpunkt 204-205°.

Beispiel 5

Zu 360 ml trockenem, kondensiertem Ammoniak werden bei ca. −33° unter Rühren 28 g (0,18 Mol) Biphenyl in 300 ml absolutem Aether zugetropft. Es wird auf ca. −50° gekühlt, worauf 2,5 g (0,36 Mol) Lithiumdraht (ca. 2 cm lange Stücke ; entfettet mit Cyclohexan) im Verlauf von 10 minuten zugegeben werden. Dann wird während 2 Stunden bei Siedetemperatur (ca. −33°) gerührt. Hierauf werden bei ca. −50° 9,4 g (0,06 Mol) (S)-2-Chlor-N,N,1-trimethyl-ëthylamin-hydrochlorid portionenweise innerhalb von ca. 3 Minuten zugegeben. Es wird während 2 Stunden bei ca. −33° gerührt und anschliessend aufgearbeitet. Nach Zugabe von Ammoniumchlorid, Abdestillieren des Ammoniaks und wässriger Aufarbeitung und Chromatographie in Analogie zu Beispiel 3 erhält man (S)-α,N,N-Trimethyl-1-phenyl-2,5-cyclohexadien-1-äthylamin, welches in üblicher Weise ins Maleat übergeführt wird. Das Produkt zeigt nach Kristallisation aus Aether den Schmelzpunkt 87-90°.

Das Ausgangsmaterial, (S)-2-Chlor-N,N,1-trimethyläthylamin-hydrochlorid wird folgendermassen erhalten :

12,4 g (0,09 Mol) (S)-2-(Dimethylamino)-1-propanolhydrochlorid werden in 100 ml Methylenchlorid gelöst und unter Eiskühlung werden 25,3 ml (0,32 Mol) Thionylchlorid innert einer halben Stunde zugetropft. Es wird während 3 Stunden bei Raumtemperatur gerührt und anschliessend am Vakuum zur Trockene eingeengt. Der so erhaltene kristalline Rückstand wird aus Methylenchlorid/Aether umkristallisiert. Das resultierende (S)-2-Chlor-N,N,1-trimethyl-äthylaminhydrochlorid zeigt einen Schmelzpunkt von 98-102°.

In gleicher Weise wird (R)-2-Chlor-N,N,1-trimethyläthylamin-hydrochlorid aus (R)-2-(Dimethylamino)-1-propanol-hydrochlorid erhalten.

In analoger Weise wie vorstehend beschrieben erhält man ausgehend von (R)-2-Chlor-N,N,1-trimethyläthylamin-hydrochlorid und Biphenyl (R)-α,N,N-Trimethyl-1-phenyl-2,5-cyclohexadien-1-äthylamin-maleat.

Beispiel 6

Zu 600 ml trockenem, kondensiertem Ammoniak werden bei −33° 15,4 g (0,1 Mol) Biphenyl in 300 ml absolutem Aether unter Rühren zugegeben. Dann werden bei −70° 1,67 g (0,24 Mol) Lithiumdraht (in ca. 2 cm lange Stücke geschnitten ; mit Cyclohexan entfettet) unter Rühren im Verlauf von 15 Minuten eingetragen. Es wird 1 Stunde bei −33° nachgerührt. Hierauf werden bei −70° 26,0 g (0,24 Mol) Chloressigsäure-methylester (frisch destilliert) in 100 ml absolutem Aether in ca. 35 Minuten eingetragen, wobei die dunkelrote Farbe des Reaktionsgemisches nach gelb umschlägt. Dann werden 13,0 g (0,24 Mol) Ammoniumchlorid eingetragen und der Ammoniak wird abdestilliert. Es werden 200 ml destilliertes Wasser zugegeben, worauf die Phasen getrennt werden. Die Aetherphase wird mit 2 N wässriger Salzsäure und mit 2 N wässriger Natriumbicarbonatlösung gewaschen. Die wässrigen Phasen werden mit Aether nachextrahiert und die vereinigten Aetherphasen werden mit Natriumchlorid und mit Natriumsulfat getrocknet, filtriert und völlig eingeengt. Vom Rückstand werden 1,5 g bei 0,03 mm Hg destilliert. Die bei 89° übergehende Fraktion enthält 1-Phenyl-2,5-cyclohexadien-1-essigsäure-methylester.

Zu einer Lösung von 11,4 g (0,3 Mol) Lithiumaluminiumhydrid in 1 150 ml absolutem Aether wird unter Rühren eine Lösung von 68,5 g (0,3 Mol) 1-Phenyl-2,5-cyclohexadien-1-essigsäure-methylester in 250 ml absolutem Aether zugetropft. Es wird 1 Stunde gerührt. Dann werden 50 ml Aethanol und anschliessend 50 ml Wasser vorsichtig zugegeben. Es wird abgenutscht und mit Aether nachgewaschen. Die Phasen werden getrennt und die vereinigten Aetherlösungen werden zur Trockene eingeengt. Man erhält eine kristallisierende Flüssigkeit, welche aus Benzol/Hexan umkristallisiert wird. Das erhaltene 1-Phenyl-2,5-cyclohexadien-1-äthanol zeigt einen Schmelzpunkt von 58-59°.

10 g (0,05 Mol) dieses Produktes werden in 70 ml trockenem DMSO gelöst. Es werden 44 ml Triäthylamin zugegeben. Dann werden 24 g (0,15 Mol) Schwefeltrioxid-Pyridin-Komplex in 100 ml trockenem Dimethylsulfoxyd unter Eiskühlung zugetropft. Nach 1 Stunde Rühren bei Raumtemperatur wird wie folgt aufgearbeitet : Das Reaktionsgemisch wird auf Wasser gegossen und zweimal mit Aether extrahiert. Die Aertherphasen werden mit 3 N wässriger Salzsäure gewaschen, mit Wasser neutral gewaschen, getrocknet, filtriert und eingeengt. Man erhält ein öliges Produkt, welches an 200 g Silicagel mit Petroläther/Aether (3/1) chromatographiert wird. Man erhält 1-Phenyl-2,5-cyclohexadien-1-acetaldehyd in Form eines öligen Produktes.

2,4 g (0,1 Mol) Magnesiumspäne werden vorgelegt und 14,2 g (0,1 Mol) Methyljodid in 15 ml absolutem Aether werden zugetropft. Es wird während 1 Stunde zum Rückfluss erhitzt, abgekühlt und über Glaswolle filtriert. Das Filtrat wird zu einer Lösung von 13 g (0,065 Mol) 1-Phenyl-2,5-cyclohexadien-1-acetaldehyd in 50 ml absolutem Aether zugetropft. Nach 3-stündigem Erhitzen zum Rückfluss wird wie folgt aufgearbeitet : Es wird ca. 2 N wässrige Ammoniumchloridlösung zugegeben und ausgeschüttelt. Die wässrige Phase wird mit Aether nachextrahiert. Die vereinigten organischen Phasen werden mit Wasser gewaschen, mit Natriumsulfat getrocknet, filtriert und eingeengt. Das Rohprodukt wird an 260 g Silicagel mit Petroläther/Aether (1/1) chromatographiert. Man erhält α-Methyl-1-phenyl-2,5-cyclohexadien-1-äthanol als öliges Produkt.

1 g (0,004 7 Mol) dieses Produktes werden in 10 ml trockenem Dimethylsulfoxyd gelöst. Es werden 3,9 ml 0,028 Mol) Triäthylamin zugegeben. Dann werden 2,3 g (0,014 5 Mol) Schwefeltrioxyd-Pyridin-Komplex in 7 ml trockenem Dimethylsulfoxyd unter Eiskühlung zugetropft. Nach 4-stündigem Rühren bei Raumtemperatur wird wie folgt aufgearbeitet : Das Reaktionsgemische wird auf Wasser gegossen und zweimal mit Aether extrahiert. Die Aetherphasen werden mit 3 N wässriger Salzsäure gewaschen, mit Wasser neutral gewaschen, getrocknet, filtriert und eingeengt. Das Rohprodukt wird mit Petroläther/Aether (1/1) an Silicagel chromatographiert. Man erhält (1-Phenyl-2,5-cyclohexadien-1-yl)-2-propanon als farbloses öliges Produkt.

Zu 50 ml Methanol, 10 ml (0,15 Mol) Dimethylamin und 1 g Molekularsieb 3A werden bei ca. −5° 10 ml 5 N methanolische Salzsäure und anschliessend bei 0° eine Lösung von 5,3 g (0,025 Mol) (1-Phenyl-2,5-cyclohexadien-1-yl)-2-propanon in 20 ml Methanol zugetropft. Dann werden bei 0° 1,1 g (0,017 5 Mol) Natriumcyanborhydrid zugegeben, und es wird während 4 Tagen bei Raumtemperatur gerührt. Das Reaktionsgemisch wird wie folgt aufgearbeitet : Dem Reaktionsgemisch wird Aether und Eiswasser zugegeben, worauf die Phasen getrennt werden. Die Aetherphase wird mit verdünnter wässriger Salzsäure extrahiert. Die sauren Auszüge werden mit konzentriertem Ammoniak alkalisch gestellt und mit Aether extrahiert. Die vereinigten Aetherphasen werden filtriert und eingeengt. Man erhält rohes α,N,N-Trimethyl-1-phenyl-2,5-cyclohexadien-1-äthylamin, das in Aether gelöst, mit Chlorwasserstoff ins Hydrochlorid übergeführt und aus Methylenchlorid/Aether umkristallisiert wird. Schmelzpunkt und Mischschmelzpunkt des so erhaltenen Produktes stimmen mit dem gemäss Beispiel 3 erhaltenen überein.

Beispiel 7

18,6 g (0,162 Mol) Methansulfonsäurechlorid werden unter Rühren und Eiskühlung zu einer Lösung

von 27 g (0,135 Mol) 1-Phenyl-2,5-cyclohexadien-1-äthanol in 270 ml absolutem Pyridin getropft. Nach 3-stündigem Rühren bei Raumtemperatur werden ca. 250 ml Wasser zugegeben. Das ausgefallene Produkt wird abgenutscht, mit Wasser gewaschen und getrocknet. Das Rohprodukt wird aus Benzol/Hexan umkristallisiert, wobei man Methansulfonsäure-2-(1-phenyl-2,5-cyclohexadien-1-yl)-äthylester als farblose Kristalle vom Schmelzpunkt 64-65° erhält.

100 g dieses Produkts werden in 600 ml Toluol gelöst. Bei −10° werden 70 ml (ca. 3 Aequivalente) kondensiertes Dimethylamin zugegeben, worauf das Reaktionsgemisch im Druckgefäss während 16 Stunden auf 150° erhitzt wird. Es wird wie folgt aufgearbeitet: Die Toluollösung wird mit Wasser gewaschen und völlig eingeengt. Das resultierende Oel wird in Benzol wieder gelöst und mit 3 N wässriger Salzsäure ausgezogen. Die wässrige Phase wird mit konzentriertem Ammoniak wieder alkalisch gestellt und mit Aether extrahiert. Die vereinigten Aetherextrakte werden mit Wasser gewaschen, mit Natriumsulfat getrocknet, filtriert und eingeengt. Man erhält rohes N,N-Dimethyl-1-phenyl-2,5-cyclohexadien-1-äthylamin, welches in üblicher Weise ins (1: 1)-Maleat übergeführt wird.

## Beispiel 8

Bei analogem Vorgehen wie in Beispiel 7 wird Methansulfonsäure-2-(1-phenyl-2,5-cyclohexadien-1-yl)-äthylester mit Methylamin umgesetzt. Das erhaltene rohe Produkt wird in üblicher Weise in das Hydrochlorid übergeführt, welches aus Isopropanol umkristallisiert wird. Man erhält N-Methyl-1-phenyl-2,5-cyclohexadien-1-äthylamin-hydrochlorid vom Schmelzpunkt 172-173°.

## Beispiel 9

87 g (0,31 Mol) Methansulfonsäure-2-(1-phenyl-2,5-cyclohexadien-1-yl)-äthylester werden in 600 ml Toluol gelöst. Es werden 95 ml (ca. 3 Aequivalente) Diäthylamin zugegeben, worauf das Reaktionsgemisch im Druckgefäss während 16 Stunden auf 150° erhitzt wird. Die Aufarbeitung erfolgt wie im zweiten Teil von Beispiel 7 beschrieben. Man erhält rohes N,N-Diäthyl-1-phenyl-2,5-cyclohexadien-1-äthylamin, welches in Aether gelöst und mit Chlorwasserstoff ins Hydrochlorid übergeführt wird. Nach Umkristallisation aus Methylenchlorid/Aether erhält man das Hydrochlorid vom Schmelzpunkt 134-136°.

## Beispiel 10

Zu 600 ml kondensiertem, über Natrium destilliertem Ammoniak werden bei −33° unter Argon 15,4 g (0,1 Mol) Biphenyl in 130 ml absolutem Aether unter Rühren zugetropft. Dann wird auf −70° gekühlt, worauf 2 g (0,288 Mol) Lithiumdraht (ca. 2 cm lange Stücke; entfettet mit Cyclohexan) während 15 Minuten eingetragen werden. Es wird 1 Stunde bei −70° gerührt, worauf 26,3 ml (0,3 Mol) frisch destillierter 2-Chlorpropionsäuremethylester in 40 ml absolutem Aether im Verlauf von 35 Minuten zugetropft werden. Nach beendeter Zugabe werden sofort 13,4 g (0,25 Mol) festes Ammoniumchlorid zugegeben. Nach Abdestillieren des Ammoniaks werden 170 ml Wasser zugetropft, worauf mit Aether extrahiert wird. Die organische Phase wird mit verdünnter Salzsäure und anschliessend mit wässriger Natriumcarbonatlösung gewaschen, mit Natriumsulfat getrocknet, filtriert und eingeengt. Das erhaltene Rohprodukt wird an 1,3 kg Silicagel mit Aether/Petroläther (1/2) chromatographiert. Man erhält 2-(1-Phenyl-2,5-cyclohexadien-1-yl)-propionsäure-methylester als öliges Produkt.

0,76 g (20 mMol) Lithiumaluminiumhydrid werden in 20 ml abs. Aether vorgelegt. 2,42 g (10 mMol) 2-(1-Phenyl-2,5-cyclohexadien-1-yl)-propionsäuremethylester in 20 ml absolutem Aether werden dazugetropft. Nach 5-stündigem Rühren bei Raumtemperatur wird wie folgt aufgearbeitet: Das Reaktionsgemisch wird mit Essigester und Wasser versetzt, abgenutscht, worauf die organische Phase von der wässrigen abgetrennt wird. Die organische Phase wird mit Natriumsulfat getrocknet, filtriert und evaporiert. Das erhaltene Rohprodukt wird an 80 g Silicagel mit Aether/Petroläther (1/2) chromatographiert, wobei man öliges β-Methyl-1-phenyl-2,5-cyclohexadien-1-äthanol erhält.

5 g (0,023 Mol) des obigen Produktes werden in 150 ml trockenem DMSO gelöst und mit 18,8 ml Triäthylamin versetzt. Hierauf werden bei Raumtemperatur 11,4 g (0,072 Mol) Schwefeltrioxid-Pyridin-Komplex in 100 ml Dimethylsulfoxyd zugetropft. Nach 2 stündiger Reaktionszeit bei Raumtemperatur wird wie folgt aufgearbeitet: Das Reaktionsgemisch wird auf Eiswasser gegossen und mit Aether extrahiert. Die organischen Phasen werden mit verdünnter wässriger Salzsäure gewaschen, mit Wasser neutral gewaschen, mit Magnesiumsulfat getrocknet, filtriert und eingeengt. Man erhält β-Methyl-1-phenyl-2,5-cyclohexadien-1-acetaldehyd in Form eines öligen Produktes.

Zu 50 ml Methanol, 6,2 ml (0,096 Mol) Dimethylamin und 1 Spatel Molekularsieb 3A werden bei 0° 2,8 ml 11,2 N methanolische Salzsäure zugetropft. Dann werden bei 0° 3,3 g (0,015 5 Mol) β-Methyl-1-phenyl-2,5-cyclohexadien-1-acetaldehyd in wenig Methanol zugetropft, worauf 0,51 g (0,081 Mol) Natriumcyanborhydrid zugegeben werden. Das Reaktionsgemisch wird während 4 Tagen bei Raumtemperatur gerührt und dann wie folgt aufgearbeitet: Das Reaktionsgemisch wird mit Aether und Bicarbonat versetzt und ausgeschüttelt. Die organische Phase wird mit verdünnter Salzsäure extrahiert. Der saure Extrakt wird mit konzentriertem Ammoniak wieder alkalisch gestellt und in Aether extrahiert. Die vereinigten organischen Phasen werden getrocknet, filtriert und völlig eingeengt. Das erhaltene

Rohprodukt wird mit Aether/Triäthylamin (10/1) an Silicagel chromatographiert, worauf das erhaltene Produkt in Aether gelöst und durch Behandeln mit Chlorwasserstoff ins Hydrochlorid übergeführt wird. Nach Kristallisation aus Methylenchlorid/Aether erhält man β,N,N-Trimethyl-1-phenyl-2,5-cyclohexadien-1-äthylamin-hydrochlorid vom Schmelzpunkt 198-199°.

Beispiel 11

Zu 1,2 l kondensiertem, trockenem Ammoniak wird eine Lösung von 30,8 g (0,2 Mol) Biphenyl in 600 ml absolutem Aether zugetropft. Dann werden portionenweise 3,47 g (0,5 Mol) Lithiumdraht (in ca. 2 cm langen Stücken ; mit Cyclohexan entfettet) zugegeben, worauf eine Stunde gerührt wird. Dann werden 73,3 g (0,49 Mol) N,N-Diäthyl-chloracetamid in 200 ml absolutem Aether zugetropft. Nach einer weiteren 1/4 Stunde Reaktionszeit wird wie folgt aufgearbeitet : Das Reaktionsgemisch wird mit 26,2 g (0,49 Mol) Ammoniumchlorid versetzt, worauf der Ammoniak abdestilliert wird. Nach Zugabe von Wasser werden die Phasen getrennt, und die organische Phase wird mit verdünnter Salzsäure und anschliessend mit 2 N Natriumcarbonatlösung in Wasser gewaschen, über Natriumsulfat getrocknet, filtriert und zur Trockene eingeengt. Der resultierende Rückstand wird durch Chromatographie über Alox gereinigt und destilliert (Sdp. 150-152° ; 0,4 mm Hg). Das erhaltene N,N-Diäthyl-1-phenyl-2,5-cyclohexadien-1-acetamid wird ohne zusätzliche Reinigung in die nächste Stufe eingesetzt.

Zu einer ätherischen Lösung von 27 g (0,1 Mol) N,N-Diäthyl-1-phenyl-2,5-cyclohexadien-1-acetamid werden 7,6 g (0,2 Mol) Lithiumaluminiumhydrid in absolutem Aether zugetropft. Nach einstündigem Rühren werden 15 ml Aethanol und anschliessend 15 ml Wasser zugegeben. Die Aetherlösung wird abdekantiert und mit verdünnter Salzsäure ausgezogen. Der saure Extrakt wird wieder alkalisch gestellt und mit Aether extrahiert. Die Aetherphasen werden getrocknet und eingeengt. Das erhaltene Oel wird destilliert (Sdp. 107-110° ; 0,1 mm Hg). Das in üblicher Weise hergestellte Hydrochlorid von N,N-Diäthyl-1-phenyl-2,5-cyclohexadien-1-äthylamin zeigt nach Kristallisation aus Isopropanol/Aether und absolutem Aethanol/Aether den Schmelzpunkt 133-134°.

Beispiel 12

8 g (0,037 Mol) α-Methyl-1-phenyl-2,5-cyclohexadien-1-äthanol werden in 50 ml Pyridin gelöst, worauf bei 0-5° 6,4 g (0,049 Mol) Mesylchlorid zugetropft werden. Nach zweistündigem Rühren bei Raumtemperatur wird wie folgt aufgearbeitet : Das Reaktionsgemisch wird mit Eiswasser versetzt und mit Aether extrahiert. Die Aetherphase wird mit 3 N wässriger Salzsäure gewaschen, dann neutral gewaschen, getrocknet und eingeengt. Das erhaltene Rohprodukt wird an 100 g Silicagel mit Aether/Petroläther (1/1) chromatographiert. Man erhält ein öliges Produkt, welches in der Kälte kristallisiert. Nach Kristallisation aus Methylenchlorid/Hexan erhält man Methansulfonsäure-1-methyl-2-(1-phenyl-2,5-cyclohexadien-1-yl)-äthylester vom Schmelzpunkt 51-53°.

Zu 1,5 g (5,14 mMol) Methansulfonsäure-1-methyl-2-(1-phenyl-2,5-cyclohexadien-1-yl)-äthylester in 5 ml Toluol werden bei −10° 1,1 ml (ca. 3 Aeq.) kondensiertes Dimethylamin gegeben. Das Reaktionsgemisch wird dann im Druckgefäss während 16 Stunden bei 150° gehalten. Anschliessend wird abgekühlt und wie folgt aufgearbeitet : Die Toluol-Phase wird mit Wasser gewaschen, eingeengt, mit Aether und 3 N wässriger Salzsäure versetzt und ausgeschüttelt. Der wässrige Auszug wird mit konzentriertem Ammoniak basisch gestellt und wieder mit Aether extrahiert. Man erhält einen öligen Rückstand, welcher an einer Dickschichtplatte mit Methylenchlorid/Methanol (10/1) chromatographiert wird. Man erhält α,N,N-Trimethyl-1-phenyl-2,5-cyclohexadien-1-äthylamin, welches in üblicher Weise in das Hydrochlorid übergeführt wird.

Beispiel 13

23 g (0,1 Mol) 1-Phenyl-2,5-cyclohexadien-1-essigsäure-methylester werden in 250 ml trockenem Tetrahydrofuran gelöst und auf ca. −70° gekühlt. Dann werden 1 g 18-Crown-6-Aether, 19 g (0,17 Mol) Kalium-tert.-butylat und 28 ml (0,45 Mol) Methyljodid zugegeben. Es wird über Nacht bei — −70° gerührt und dann wie folgt aufgearbeitet : Das Reaktionsgemisch wird auf Eiswasser gegossen und mit Aether extrahiert. Die organischen Phasen werden mit Wasser gewaschen, mit Magnesiumsulfat getrocknet, filtriert une völlig eingeengt. Das resultierende Oel wird über eine Silicagelsäule filtriert. Das so erhaltene Produkt (2-(1-Phenyl-2,5-cyclohexadien-1-yl)-propionsäure-methylester) wird ohne weitere Reinigung in die nächste Stufe eingesetzt.

3 g (0,079 Mol) Lithiumaluminiumhydrid werden in 100 ml trockenem Aether vorgelegt und 18,9 g des gemäss oben erhaltenen Produktes werden in 80 ml Aether dazugetropft. Nach zweistündiger Reaktionszeit bei Raumtemperatur wird wie folgt aufgearbeitet : Das überschüssige Lithiumaluminiumhydrid wird mit Alkohol und anschliessend mit Wasser zersetzt. Nach Filtrieren werden die Phasen getrennt und die Aetherphase wird mit Wasser gewaschen, mit Magnesiumsulfat getrocknet, filtriert und eingeengt. Man erhält ein klares Oel, welches mit Petroläther/Aether (1/1) an Silicagel chromatographiert wird. Man erhält β-Methyl-1-phenyl-2,5-cyclohexadien-1-äthanol als öliges Produkt.

15 g (70 mMol) des obigen Produktes werden in 200 ml Pyridin gelöst, worauf 8,8 ml (113 mMol)

Methansulfonsäurechlorid eingetropft werden. Nach 6-stündigem Rühren bei Raumtemperatur wird wie folgt aufgearbeitet: Das Reaktionsgemisch wird mit Aether versetzt und mit Wasser, 3 N wässriger Salzsäure, wieder mit Wasser gewaschen, über Natriumsulfat getrocknet, filtriert und zur Trockene eingeengt. Das erhaltene Rohprodukt wird an Silicagel mit Petroläther/Aether (1/2) chromatographiert, wobei man öligen Methansulfonsäure-2-(1-phenyl-2,5-cyclohexadien-1-yl)-propylester erhält.

9,4 g (32,2 mMol) dieses Produktes werden in 8 ml Toluol gelöst. Bei $-10°$ werden 4,1 ml (ca. 3 Aeq.) kondensiertes Methylamin zugegeben. Das Reaktionsgemisch wird dann im Druckgefäss während 24 Stunden bei 150° gehalten. Anschliessend wird erneut mit 8,2 ml Methylamin versetzt und das Reaktionsgemisch weitere 24 Stunden bei 150° gehalten. Anschliessend wird das Reaktionsgemisch eingeengt, mit Aether und 3 N Salzsäurere versetzt und ausgeschüttelt. Nach Abnutschen der ausgefallenen Kristalle wird die wässrige Phase wieder alkalisch gestellt und mit Methylenchlorid extrahiert. Die Kristallfraktion wird ebenfalls mit Methylenchlorid und wässriger Natronlauge behandelt. Die vergnigten Methylenchloridphasen werden mit Wasser neutral gewaschen, mit Natriumsulfat getrocknet, filtriert und eingeengt. Das erhaltene ölige Produkt wird mit Methylenchlorid/Methanol (10/1) and 120 g Alox (neutral) chromatographiert. Man erhält β-N-Dimethyl-1-phenyl-2,5-cyclohexadien-1-äthylamin. Das in üblicher Weise hergestellte Hydrochlorid zeigt den Schmelzpunkt $> 250°$.

Beispiel 14

600 ml über Natrium destillierter Ammoniak werden unter Argon bei $-33°$ vorgelegt. Hierauf werden 15,4 g (0,1 Mol) Biphenyl in 130 ml Aether unter Rühren zugetropft. Nach Kühlen auf $-70°$ werden 2 g Lithium-Draht (in ca. 2 cm-Stücke geschnitten; mit Cyclohexan entfettet) im Verlauf von 10 Minuten zugegeben. Nach einstündigem Rühren bei $-70°$ werden 18,6 g (0,25 Mol) frisch destilliertes Chloracetonitril in 45 ml absolutem Aether im Verlauf von 45 Minuten zugetropft. Anschliessend werden sofort 13,4 g (0,25 Mol) festes Ammoniumchlorid zugegeben, worauf der Ammoniak abdestilliert wird. Das Reaktionsgemisch wird auf Eiswasser gegossen und mit Aether extrahiert. Die organische Phase wird mit verdünnter Salzsäure und anschliessend mit wässriger Natriumcarbonatlösung gewaschen, getrocknet, filtriert und eingeengt. Das Rohprodukt wird an 1 kg Silicagel mit Aether/Petroläther (1/2) chromatographiert. Man erhält reines 1-Phenyl-2,5-cyclohexadien-1-acetonitril.

0,68 g (5,1 mMol) Aluminiumtrichlorid und 0,24 g (6,3 mMol) Lithiumaluminiumhydrid werden in 8 ml absolutem Aether vorgelegt. Unter Rühren werden 1 g (5,1 mMol) 1-Phenyl-2,5-cyclohexadien-1-acetonitril in 5 ml absolutem Aether zugetropft. Nach 2-stündigem Rühren bei Raumtemperatur wird wie folgt aufgearbeitet: Das Reaktionsgemisch wird mit 1 ml Wasser und 25 ml 3 N wässriger Natronlauge versetzt, abgenutscht und mit Aether nachgespült. Nach der Phasentrennung wird die organische Phase über Natriumsulfat getrocknet, filtriert und zur Trockene eingeengt. Das erhaltene Rohprodukt wird an 50 g Alox (neutral) chromatographiert, wobei mit Aether vorerst Edukt und dann mit Methylenchlorid/Methanol (10/1) Endprodukt eluiert wird. Man erhält 1-Phenyl-2,5-cyclohexadien-1-äthylamin als öliges Produkt. Ein Teil dieses Produktes wird auf übliche Weise ins Hydrochlorid übergeführt, welches nach Umkristallisation aus Methylenchlorid/Aether den Schmelzpunkt 170-172° zeigt.

4 g (17 mMol) 1-Phenyl-2,5-cyclohexadien-1-äthylamin-hydrochlorid werden in 100 ml absolutem Methanol vorgelegt. Nach Zugabe von 3,3 g (6,5 Aeq.) p-Formaldehyd, 1 g (ca. 1 Aeq.) Natriumcyanborhydrid und 5 g Molekularsieb 3A wird über Nacht bei Raumtemperatur gerührt und anschliessen wie folgt aufgearbeitet: Das Reaktionsgemisch wird auf wässrige 3 N Salzsäure gegossen, 10 Minuten gerührt, mit konzentriertem wässrigem Ammoniak basisch gestellt, mit Essigester extrahiert, mit Wasser gewaschen, mit Natriumsulfat getrocknet und filtriert. Man erhält ein öliges Produkt, welches mit Methylenchlorid/Methanol (10/1) an 80 g Silicagel chromatographiert wird. Das erhaltene gelbe Oel (N,N-Dimethyl-1-phenyl-2,5-cyclohexadien-1-äthylamin) wird in üblicher Weise ins (1: 1)-Maleat übergeführt, welches den Schmelzpunkt 129-131° zeigt.

Beispiel 15

Zu 6,4 g (8,6 Aeq.) Dimethylamin und 5 g Molekularsieb 3A in 50 ml absolutem Methanol werden bei 0° 6,6 ml 5 N methanolische Salzsäure (2 Aeq.) und anschliessend 3,3 g (16,6 mMol) 1-Phenyl-2,5-cyclohexadien-1-acetaldehyd in 10 ml absolutem Methanol zugetropft. Nach Zugabe von 0,75 g (0,72 Aeq.) Natriumcyanborhydrid wird während 5 Tagen bei Raumtemperatur gerührt. Das Reaktionsgemisch wird mit Eis/Wasser und Aether versetzt und mit 3 N wässriger Salzsäure extrahiert. Die salzsauren Extrakte werden mit konzentriertem wässrigem Ammoniak basisch gestellt und wieder mit Aether extrahiert. Die Aether-Phase wird mit Natriumsulfat getrocknet, filtriert, evaporiert und an Alox neutral chromatographiert. Das so erhaltene N,N-Dimethyl-1-phenyl-2,5-cyclohexadien-1-äthylamin wird wie üblich in das (1: 1)-Maleat übergeführt, welches den Schmelzpunkt 128-130° zeigt.

Beispiel 16

Zu 11,5 ml (ca. 6 Aeq.) Methylamin unf 10 g Molekularsieb 3A in 200 ml absolutem Methanol werden

bei 0° 17,8 ml 5 N methanolische Salzsäure (2 Aeq.) und anschliessend 9,4 g (44 mMol) (1-Phenyl-2,5-cyclohexadien-1-yl)-2-propanon in wenig Methanol zugetropft. Nach Zugabe von 1,95 g (0,75 Aeq.) Natriumcyanborhydrid wird 5 Tage bei Raumtemperatur gerührt. Das Reaktionsgemisch wird wie folgt aufgearbeitet : Es wird mit Eis/Wasser und Aether versetzt und mit 3 N wässriger Salzsäure extrahiert. Die salzsauren Extrakte werden mit konzentriertem wässrigem Ammoniak basisch gestellt und wieder mit Aether extrahiert. Die Aether-Phase wird mit Natriumsulfat getrocknet, filtriert und evaporiert. Das erhaltene Rohprodukt wird mit Methylenchlorid/Methanol (10/1) an Silicagel chromatographiert. Man erhält α,N-Dimethyl-1-phenyl-2,5-cyclohexadien-1-äthylamin in Form eines öligen Produktes. Dieses wird wie üblich ins Hydrochlorid übergeführt, welches nach Kristallisation aus Methylenchlorid/Aether den Schmelzpunkt 155-158° zeigt.

Beispiel 17

Zu 5,3 g (ca. 8 Aeq.) Aethylamin und 10 g Molekularsieb 3A in 100 ml absolutem Methanol werden bei 0° 5,6 ml 5 N methanolische Salzsäure (2 Aeq.) und dann 3 g (14,1 mMol) (1-Phenyl-2,5-cyclohexadien-1-yl)-2-propanon in wenig absolutem Methanol zugegeben. Dann werden 0,63 g (0,7 Aeq.) Natriumcyanborhydrid zugegeben und es wird während 5 Tagen bei Raumtemperatur gerührt. Es wird völlig analog wie im Beispiel 16 beschrieben aufgearbeitet. Das Rohprodukt wird mit Methylenchlorid/Methanol (10/1) an Silicagel chromatographiert. Man erhält α-Methyl-N-äthyl-1-phenyl-2,5-cyclohexadien-1-äthylamin als öliges Produkt. Dieses Produkt wird wie üblich ins Hydrochlorid übergeführt, welches nach Kristallisation aus Methylenchlorid/Aether den Schmelzpunkt 151-153° zeigt.

Beispiel 18

1,5 g (6,2 mMol) α-Methyl-N-äthyl-1-phenyl-2,5-cyclohexadien-1-äthylamin werden in einer Mischung von 10 ml Essigsäureanhydrid und 8 ml Pyridin gelöst und über Nacht bei Raumtemperatur stehengelassen. Dann wird während 2 Stunden mit 2 N wässriger Kaliumbicarbonatlösung gerührt und anschliessend mit Aether extrahiert. Die Aether-Phase wird mit verdünnter wässriger Salzsäure und dann mit Wasser gewaschen, mit Natriumsulfat getrocknet, filtriert und zur Trockene eingeengt. Das erhaltene Rohprodukt wird mit Aether an Silicagel chromatographiert. Man erhält N-Aethyl-N-[1-methyl-2-(1-phenyl-2,5-cyclohexadien-1-yl) äthyl]-acetamid in Form eines öligen Produktes.

0,266 g (7 mMol) Lithiumaluminiumhydrid werden in 10 ml Tetrahydrofuran vorgelegt. Nach Zugabe von 1 g (3,5 mMol) N-Aethyl-N-[1-methyl-2-(1-phenyl-2,5-cyclohexadien-1-yl)-äthyl]-acetamid in 10 ml Tetrahydrofuran wird während 7 Stunden bei Raumtemperatur gerührt. Anschliessend wird überschüssiges Lithiumaluminiumhydrid durch Zugabe von Essigester und von Wasser zersetzt. Das Reaktionsgemisch wird filtriert, wobei mit Aether nachgespült wird. Nach Trennen der Phasen wird die organische Phase mit Natriumsulfat getrocknet, filtriert und völlig eingeengt. Das Rohprodukt wird an 60 g Alox neutral mit Essigester chromatographiert. Man erhält α-Methyl-N,N-diäthyl-1-phenyl-2,5-cyclohexadien-1-äthylamin in Form eines öligen Produktes. Dieses wird wie üblich ins Hydrochlorid übergeführt, welches nach Kristallisation aus Methylenchlorid/Aether den Schmelzpunkt 172-174° zeigt.

Beispiel 19

8 g (27,4 mMol) Methansulfonsäure-2-(1-phenyl-2,5-cyclohexadien-1-yl)-propylester werden in 10 ml Toluol gelöst. Bei −10° werden 8,4 ml (ca. 3 Aeq.) kondensiertes Diäthylamin zugegeben. Das Reaktionsgemisch wird anschliessend im Druckgefäss während 25 Stunden bei 150° gehalten. Nach dem Abkühlen wird die Toluol-Phase mit Wasser gewaschen, eingeengt, mit Aether und 3 N wässriger Salzsäure versetzt und ausgeschüttelt. Die wässrige Phase wird mit konzentriertem wässrigem Ammoniak basisch gestellt und wieder mit Aether extrahiert. Die vereinigten Aether-Phasen werden über Natriumsulfat getrocknet, filtriert und völlig eingeengt. Man erhält ein öliges Produkt, welches an Silicagel mit Methylenchlorid/Methanol (10/1) chromatographiert wird. Man erhält β-Methyl-N,N-diäthyl-1-phenyl-2,5-cyclohexadien-1-äthylamin als öliges Produkt. Dieses Produkt wird wie üblich ins Hydrochlorid übergeführt, welches nach Umkristallisation aus Aethanol/Aether den Schmelzpunkt 132-137° aufweist.

Beispiel 20

Zu 27,5 ml (ca. 6 Aeq.) Diäthylamin und 10 g Molekularsieb 3A in 200 ml Methanol werden bei 0° 17,6 ml 5 N methanolische Salzsäure (2 Aeq.) und dann 9,4 g (44 mMol) (1-Phenyl-2,5-cyclohexadien-1-yl)-2-propanon in wenig Methanol und schliesslich 1,95 g (0,7 Aeq.) Natriumcyanborhydrid zugegeben. Das Reaktionsgemisch wird während 5 Tagen bei Raumtemperatur gerührt, worauf völlig analog wie im Beispiel 16 beschrieben aufgearbeitet wird. Man erhält einen öligen Rückstand welcher mit Essigester/Triäthylamin (15/1) an einer Silicagel-Dickschichtplatte chromatographiert wird. Es werden 2 Produkte abgekratzt und mit Methylenchlorid/Methanol (4/1) eluiert. Das Produkt vom RF 0.32 besteht aus α-Methyl-N-äthyl-1-phenyl-2,5-cyclohexadien-1-äthylamin. Das andere Produkt (RF 0,54) entspricht dem gewünschten Endprodukt, nämlich α-Methyl-N,N-diäthyl-1-phenyl-2,5-cyclohexadien-1-äthylamin. Dieses

wird wie üblich ins Hydrochlorid übergeführt und aus Methylenchlorid/Aether umkristallisiert. Schmelzpunkt und Mischschmelzpunkt sind mit dem gemäss Beispiel 18 erhaltenen Material in Uebereinstimmung.

Beispiel 21

Zu 600 ml über Natrium destilliertem Ammoniak werden bei −33° unter Argon 46 g (0,3 Mol) Biphenyl in 400 ml Aether zugetropft. Nach Abkühlen auf −50° werden 12 g (0,3 Mol) granuliertes Calcium während 15 Minuten zugegeben. Die Lösung wird grün-gelb und schliesslich dunkelrot bis schwarz. Es wird während 2 Studen bei −33° gerührt und dann auf −70° gekühlt. Dem Reaktionsgemisch werden während 5 Minuten 14,4 g (0,11 Mol) 2-Chlor-N,N-dimethyläthylamin-hydrochlorid zugegeben. Nach 2-stündigem Rühren bei −33° wird wieder auf −70° gekühlt, worauf 32 g (0,6 Mol) Ammonchlorid zugegeben werden. Der Ammoniak wird über Nacht abdestilliert. Das Reaktionsgemisch wird mit 400 ml Wasser versetzt und dann durch Zugabe von 28 %-iger wässriger Natronlauge stark basisch gestellt. Nach dem Abfiltrieren werden die Phasen getrennt, und die organische Phase wird mit 3 N wässriger Salzsäure extrahiert. Der wässrig saure Extrakt wird mit Natronlauge wieder basisch gestellt und mit Aether extrahiert. Die Aether-Phase wird mit Natriumsulfat getrocknet, filtriert und zur Trockene eingeengt. Man erhält ein öliges Produkt, welches mit Methylenchlorid/Methanol (10/1) an 200 g Silicagel chromatographiert wird. Das erhaltene reine N,N-Dimethyl-1-phenyl-2,5-cyclohexadien-1-äthylamin wird wie üblich ins (1: 1)-Maleat übergeführt. Das so erhaltene Maleat schmilzt bei 128-130°.

Die nachfolgenden Beispiele illustrieren galenische Formulierungen.

Beispeil A

Hartgelatine-Kapsel :

a) Zusammensetzung :

| | |
|---|---|
| N,N-Dimethyl-1-phenyl-2,5-cyclohexadien-1-äthylamin-maleat | 100,0 mg |
| Milchzucker krist. | 102,0 mg |
| Maisstärke weiss | 45,0 mg |
| Talk | 10,4 mg |
| Magnesiumstearat | 2,6 mg |
| | 260,0 mg |

b) Herstellung :

Der Wirkstoff, wird mit Maisstärke, Talk und Magnesiumstearat gemischt, das Gemisch gesiebt, mit Milchzucker versetzt, gemischt und nochmals gesiebt. Die Pulvermischung wird in Kapseln der Grösse 1 abgefüllt.

Beispiel B

Tablette :

a) Zusammensetzung :

| | |
|---|---|
| N,N-Dimethyl-1-phenyl-2,5-cyclohexadien-1-äthylamin-maleat | 300,0 mg |
| Milchzucker krist. | 75,0 mg |
| Maisstärke weiss | 60,0 mg |
| Primojel R | 12,0 mg |
| Magnesiumstearat | 3,0 mg |
| | 450,0 mg |

b) Herstellung :

Der Wirkstoff wird mit Milchzucker und einem Teil der Maisstärke gemischt, das Gemisch mit Maisstärke-Wasser-Kleister verarbeitet, granuliert, getrocknet und gesiebt. Das Granulat wird mit Primojel und Magnesiumstearat gemischt und zu Tabletten à 450 mg verpresst.

**Ansprüche** (für die Vertragsstaaten : BE, CH, DE, FR, GB, IT, LU, NL, SE)

1. Cyclohexadien-Derivate der allgemeinen Formel

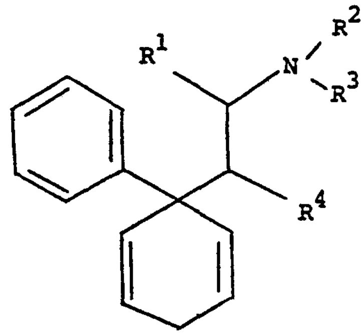

(I)

worin $R^1$ Wasserstoff oder Methyl, $R^2$ Wasserstoff, Methyl oder Aethyl, $R^3$ Methyl oder Aethyl und $R^4$ Wasserstoff oder Methyl bedeuten, wobei von den Resten $R^1$ und $R^4$ mindestens einer Wasserstoff bedeutet (einschliesslich der optisch aktiven Antipoden solcher Verbindungen, die ein asymmetrisches Kohlenstoffatom aufweisen), sowie Säureadditionssalze davon.

2. N,N-Dimethyl-1-phenyl-2,5-cyclohexadien-1-äthylamin und dessen pharmazeutisch verträgliche Säureadditionssalze.

3. Cyclohexadien-Derivate gemäss Anspruch 1 zur Verwendung als pharmazeutische Wirkstoffe.

4. Cyclohexadien-Derivate gemäss Anspruch 3 zur Verwendung als analgetische Wirkstoffe.

5. N,N-Dimethyl-1-phenyl-2,5-cyclohexadien-1-äthylamin oder ein pharmazeutisch verträgliches Säureadditionssalz davon gemäss Anspruch 3 Verwendung als pharmazeutischer Wirkstoff.

6. Cyclohexadien-Derivat gemäss Anspruch 5 zur Verwendung als analgetischer Wirkstoff.

7. Verfahren zur Herstellung von Cyclohexadien-Derivatengemäss Anspruch 1, dadurch gekennzeichnet, dass man

a) Biphenyl in Gegenwart von Lithium oder Calcium und Ammoniak mit einer Verbindung der allgemeinen Formel

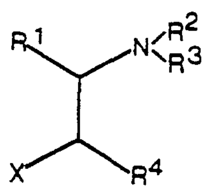

(II)

(gegebenenfalls in Form eines optischen Antipoden und/oder Säureadditionssalzes), worin $R^1$, $R^2$, $R^3$ und $R^4$ die genannte Bedeutung haben, und X für eine Abgangsgruppe steht,
umsetzt, oder

b) eine Verbindung der allgemeinen Formel

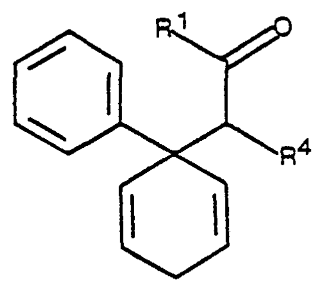

(III)

worin $R^1$ und $R^4$ die genannte Bedeutung haben, unter reduzierenden Bedingungen mit einem Amin der allgemeinen Formel

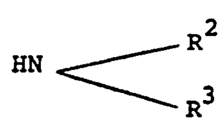

(IV)

worin $R^2$ und $R^3$ die genannte Bedeutung haben,
umsetzt, oder

c) eine Verbindung der allgemeinen Formel

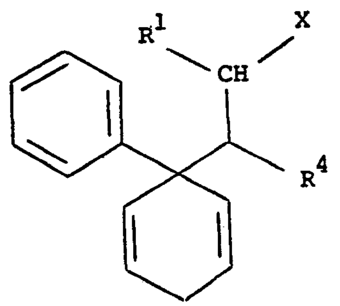

(V)

worin $R^1$ und $R^4$ die genannte Bedeutung haben und X für eine Abgangsgruppe steht, mit einem Amin der Formel IV umsetzt, oder

d) eine Verbindung der allgemeinen Formel

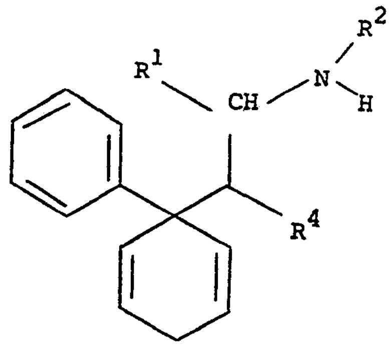

(VI)

worin $R^1$, $R^2$ und $R^4$ die genannte Bedeutung haben, entsprechend alkyliert, oder

e) eine Verbindung der allgemeinen Formel

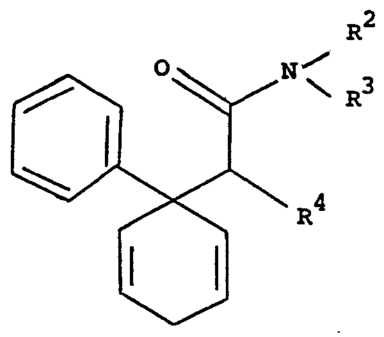

(VII)

worin $R^2$, $R^3$ und $R^4$ die genannte Bedeutung haben, reduziert, worauf man gewünschtenfalls ein erhaltenes Racemat in die optisch aktiven Antipoden aufspaltet und ein erhaltenes Produkt gewünschtenfalls in ein Säureadditionssalz überführt.

8. Arzneimittel enthaltend ein Cyclohexadien-Derivat gemäss Anspruch 1.

9. Arzneimittel gemäss Anspruch 8 als Analgetikum.

**Ansprüche** (für den Vertragsstaat AT)

1. Verfahren zur Herstellung von Cyclohexadien-Derivaten der allgemeinen Formel

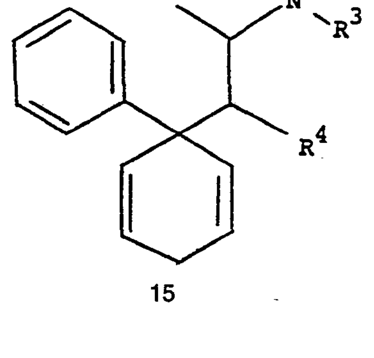

(I)

worin $R^1$ Wasserstoff oder Methyl, $R^2$ Wasserstoff, Methyl oder Aethyl, $R^3$ Methyl oder Aethyl und $R^4$ Wasserstoff oder Methyl bedeuten, wobei von den Resten $R^1$ und $R^4$ mindestens einer Wasserstoff bedeutet (einschliesslich der optisch aktiven Antipoden solcher Verbindungen, die ein asymmetrisches Kohlenstoffatom aufweisen), sowie von Säureadditionssalzen davon, dadurch gekennzeichnet, dass man

a) Biphenyl in Gegenwart von Lithium oder Calcium und Ammoniak mit einer Verbindung der allgemeinen Formel

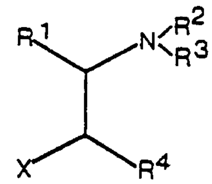

(II)

(gegebenenfalls in Form eines optischen Antipoden und/oder Säureadditionssalzes) worin $R^1$, $R^2$, $R^3$ und $R^4$ die genannte Bedeutung haben, und X für eine Abgangsgruppe steht,
umsetzt, oder

b) eine Verbindung der allgemeinen Formel

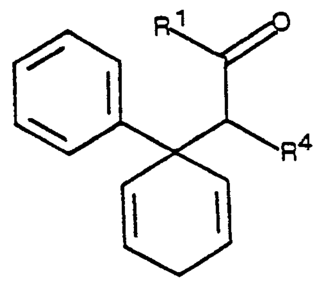

(III)

worin $R^1$ und $R^4$ die genannte Bedeutung haben, unter reduzierenden Bedingungen mit einem Amin der allgemeinen Formel

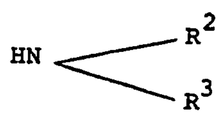

(IV)

worin $R^2$ und $R^3$ die genannte Bedeutung haben, umsetzt, oder

c) eine Verbindung der allgemeinen Formel

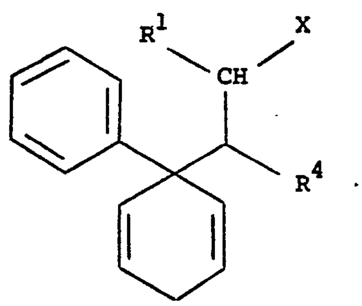

(V)

worin $R^1$ und $R^4$ die genannte Bedeutung haben und X für eine Abgangsgruppe steht, mit einem Amin der Formel IV umsetzt, oder

d) eine Verbindung der allgemeinen Formel

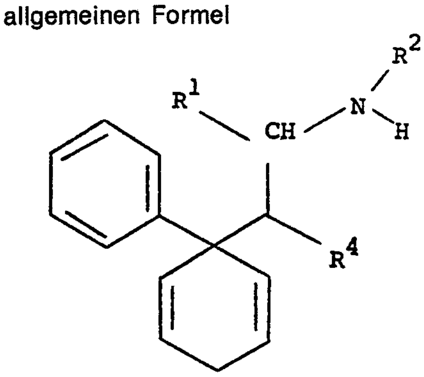

(VI)

worin $R^1$, $R^2$ und $R^4$ die genannte Bedeutung haben, entsprechend alkyliert, oder

e) eine Verbindung der allgemeinen Formel

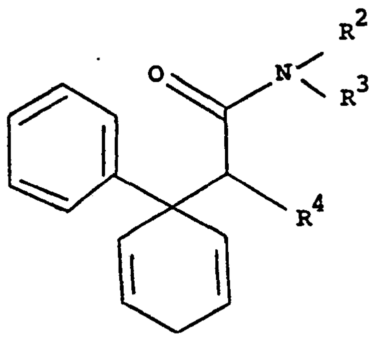

(VII)

worin $R^2$, $R^3$ und $R^4$ die genannte Bedeutung haben, reduziert, worauf man gewünschtenfalls ein erhaltenes Racemat in die optisch aktiven Antipoden aufspaltet und ein erhaltenes Produkt gewünschtenfalls in ein Säureadditionssalz überführt.

2. Verfahren nach Anspruch 1 zur Herstellung von N,N-Dimethyl-1-phenyl-2,5-cyclohexadien-1-äthylamin oder einem pharmazeutisch verträglichen Säureadditionssalz davon, dadurch gekennzeichnet, dass man entsprechend substituierte Ausgangsstoffe einsetzt und gegebenenfalls eine erhaltene freie Base in ein pharmazeutisch verträgliches Säureadditionssalz überführt.

**Claims** (for the contracting States : BE, CH, DE, FR, GB, IT, LU, NL, SE)

1. Cyclohexadiene derivatives of the general formula

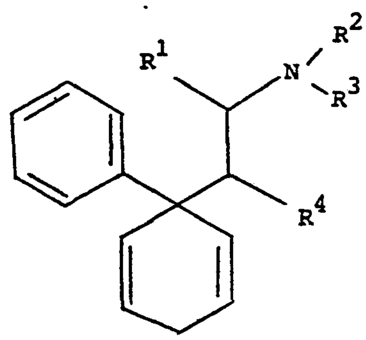

(I)

wherein $R^1$ signifies hydrogen or methyl, $R^2$ signifies hydrogen, methyl or ethyl, $R^3$ signifies methyl or ethyl and $R^4$ signifies hydrogen or methyl, whereby of the residues $R^1$ and $R^4$ at least one signifies hydrogen (including the optically active antipodes of those compounds which have an asymmetric carbon atom),
as well as acid addition salts thereof.

2. N,N-Dimethyl-1-phenyl-2,5-cyclohexadien-1-ethylamine and its pharmaceutically compatible acid addition salts.

3. Cyclohexadiene derivatives according to claim 1 for use as pharmaceutically active substances.

4. Cyclohexadiene derivatives according to claim 3 for use as analgesically active substances.

5. N,N-Dimethyl-1-phenyl-2,5-cyclohexadien-1-ethylamine or a pharmaceutically compatible acid addition salt thereof according to claim 3 for use as a pharmaceutically active substance.

6. A cyclohexadiene derivative according to claim 5 for use as an analgesically active substance.

7. A process for the manufacture of cyclohexadiene derivatives according to claim 1, characterized by

a) reacting biphenyl in the presence of lithium or calcium and ammonia with a compound of the general formula

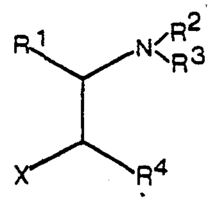

(II)

(optionally in the form of an optical antipode and/or an acid addition salt), wherein $R^1$, $R^2$, $R^3$ and $R^4$ have the above-mentioned significance, and X stands for a leaving group,
or

b) reacting a compound of the general formula

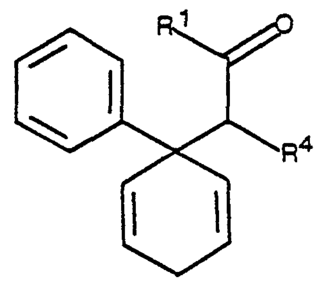

(III)

wherein $R^1$ and $R^4$ have the above-mentioned significance, under reducing conditions with an amine of the general formula

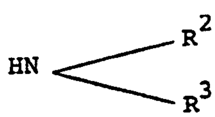

(IV)

wherein $R^2$ and $R^3$ have the above-mentioned significance,
or

c) reacting a compound of the general formula

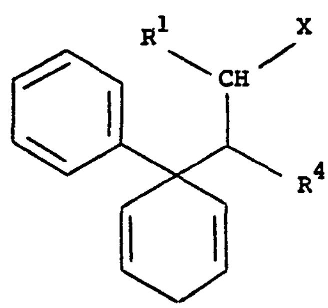

(V)

wherein $R^1$ and $R^4$ have the above-mentioned significance and X stands for a leaving group, with an amine of formula IV, or

d) appropriately alkylating a compound of the general formula

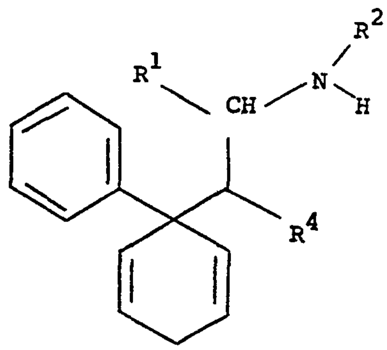

(VI)

wherein $R^1$, $R^2$ and $R^4$ have the above-mentioned significance,
or

e) reducing a compound of the general formula

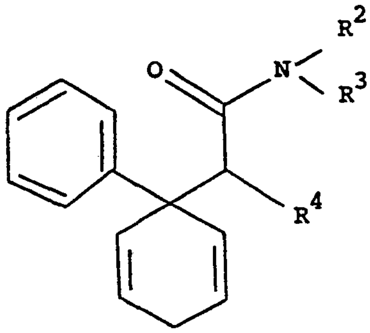

(VII)

wherein $R^2$, $R^3$ and $R^4$ have the above-mentioned significance, whereupon, if desired a racemate obtained is resolved into the optically active antipodes and a product obtained is converted into an acid addition salt if desired.

8. A medicament containing a cyclohexadiene derivative according to claim 1.

9. A medicament according to claim 8 as an analgesic.

**Claims** (for the contracting State AT)

1. A process for the manufacture of cyclohexadiene derivatives of the general formula

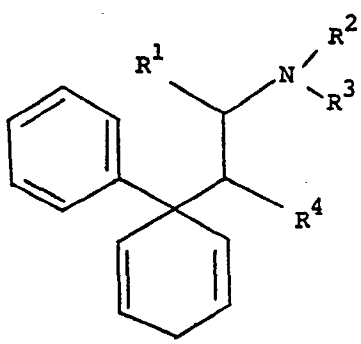

(I)

wherein $R^1$ signifies hydrogen or methyl, $R^2$ signifies hydrogen, methyl or ethyl, $R^3$ signifies methyl or ethyl and $R^4$ signifies hydrogen or methyl, whereby of the residues $R^1$ and $R^4$ at least one signifies hydrogen (including the optically active antipodes of those compounds which have an asymmetric carbon atom), as well as of acid addition salts thereof, characterized by :

a) reacting biphenyl in the presence of lithium or calcium and ammonia with a compound of the general formula

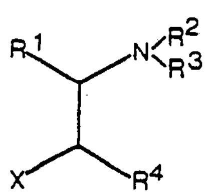

(II)

(optionally in the form of an optical antipode and/or an acid addition salt), wherein $R^1$, $R^2$, $R^3$ and $R^4$ have the above-mentioned significance, and X stands for a leaving group,
or

b) reacting a compound of the general formula

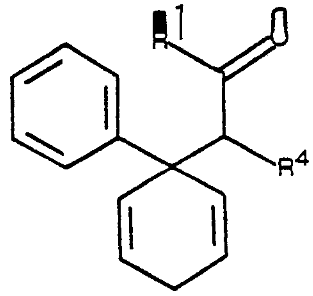

(III)

wherein $R^1$ and $R^4$ have the above-mentioned significance, under reducing conditions with an amine of the general formula

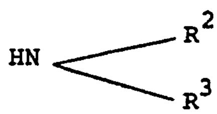

(IV)

wherein $R^2$ and $R^3$ have the above-mentioned significance, or

c) reacting a compound of the general formula

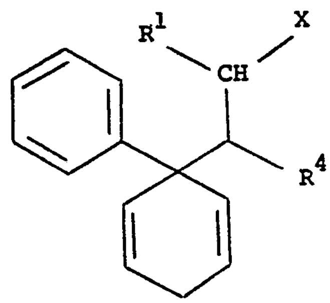

(V)

wherein $R^1$ and $R^4$ have the above-mentioned significance and X stands for a leaving group, with an amine of formula IV, or

d) appropriately alkylating a compound of the general formula

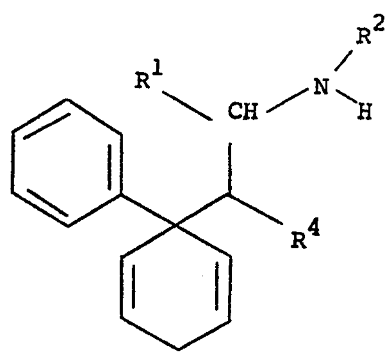

(VI)

wherein $R^1$, $R^2$ and $R^4$ have the above-mentioned significance, or

e) reducing a compound of the general formula

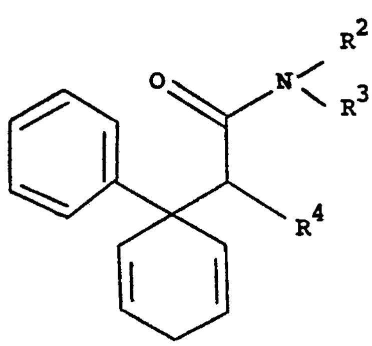

(VII)

wherein $R^2$, $R^3$ and $R^4$ have the above-mentioned significance, whereupon, if desired a racemate obtained is resolved into the optically active antipodes and a product obtained is converted into an acid addition salt if desired.

2. A process according to Claim 1 for the manufacture of N,N-dimethyl-1-phenyl-2,5-cyclohexadien-1-ethylamine or a pharmaceutically compatible acid addition salt thereof, characterized by using correspondingly substituted starting materials and, if desired, converting the free base obtained into a pharmaceutically compatible acid addition salt.

**Revendications** (pour les Etats contractants : BE, CH, DE, FR, GB, IT, LU, NL, SE)

1. Dérivés de cyclohexadiène de formule générale

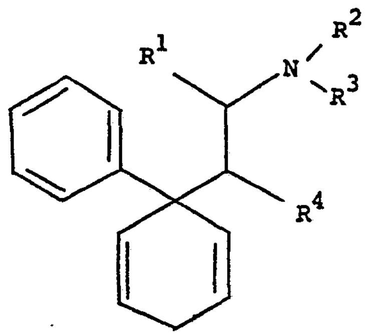

(I)

où $R^1$ représente un hydrogène ou un méthyle, $R^2$ un hydrogène, un méthyle ou un éthyle, $R^3$ un méthyle ou un éthyle, et $R^4$ un hydrogène ou un méthyle, où au moins l'un des radicaux $R^1$ et $R^4$ représente un hydrogène (y compris les antipodes optiquement actifs de ces composés, qui présentent un atome de carbone asymétrique), ainsi que leurs sels d'addition d'acides.

2. La N,N-diméthyl-1-phényl-2,5-cyclohexadièn-1-éthylamine et ses sels d'addition d'acides pharmaceutiquement acceptables.

3. Les dérivés de cyclohexadiène selon la revendication 1 en tant que substances actives pharmaceutiques.

4. Les dérivés de cyclohexadiène selon la revendication 3 en tant que substances analgésiques.

5. La N,N-diméthyl-1-phényl-2,5-cyclohexadièn-1-éthylamine ou un sel d'addition d'acide pharmaceutiquement acceptable de ce composé selon la revendication 3 en tant que substance active pharmaceutique.

6. Un dérivé de cyclohexadiène selon la revendication 5 en tant que substance analgésique.

7. Procédé de préparation de dérivés de cyclohexadiène selon la revendication 1, caractérisé en ce que :

a) On fait réagir du diphényle en présence de lithium ou de calcium et d'ammoniac avec un composé de formule générale

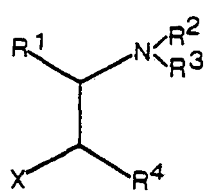

(II)

(éventuellement sous la forme d'un antipode optique et/ou d'un sel d'addition d'acide (où $R^1$, $R^2$, $R^3$ et $R^4$ ont la signification mentionnée et X représente un groupe séparable, ou

b) On fait réagir un composé de formule générale

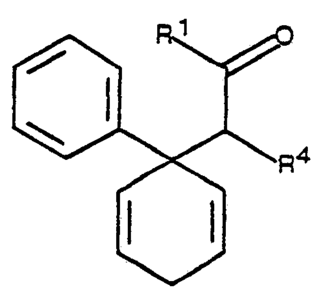

(III)

où $R^1$ et $R^4$ ont la signification mentionnée, dans des conditions réductrices avec une amine de formule générale

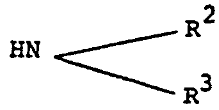

(IV)

où $R^2$ et $R^3$ ont la signification mentionnée, ou

c) On fait réagir un composé de formule générale

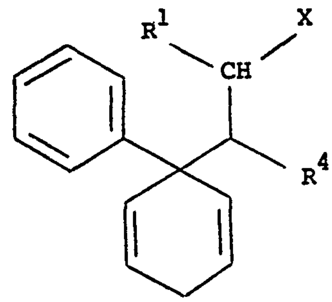

(V)

où $R^1$ et $R^4$ ont la signification mentionnée et où X représente un groupe séparable avec une amine de formule IV, ou

d) On alcoyle de façon correspondante un composé de formule générale

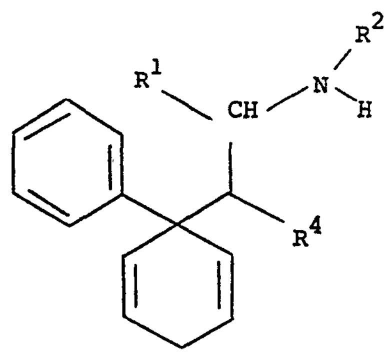

(VI)

où $R^1$, $R^2$ et $R^4$ ont la signification mentionnée, ou

e) On réduit un composé de formule générale

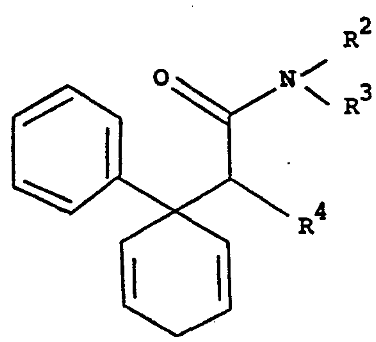

(VII)

où $R^2$, $R^3$ et $R^4$ ont la signification mentionnée, puis, si on le désire, on dédouble le racémate obtenu en ses antipodes optiquement actifs et on transforme éventuellement un produit obtenu en un sel d'addition d'acide.

8. Médicament contenant un dérivé de cyclohexadiène selon la revendication 1.

9. Médicament selon la revendication 8 en tant qu'analgésique.

**Revendications** (pour l'Etat contractant AT)

1. Procédé de préparation de dérivés de cyclohexadiène de formule générale

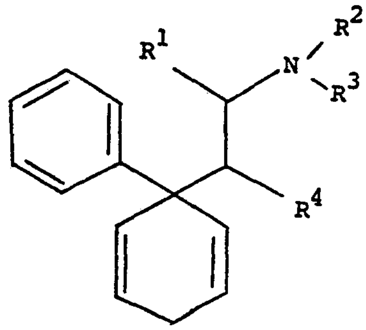

(I)

où $R^1$ représente un hydrogène ou un méthyle, $R^2$ un hydrogène, un méthyle ou un éthyle, $R^3$ un méthyle ou un éthyle, et $R^4$ un hydrogène ou un méthyle, où au moins l'un des radicaux $R^1$ et $R^4$ représente un hydrogène (y compris les antipodes optiquement actifs de ces composés, qui présentent un atome de carbone asymétrique), ainsi que de leurs sels d'addition d'acides, caractérisé en ce que :

a) On fait réagir du diphényle en présence de lithium ou de calcium et d'ammoniac avec un composé de formule générale

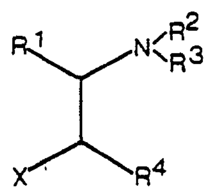

(II)

(éventuellement sous la forme d'un antipode optique et/ou d'un sel d'addition d'acide) où $R^1$, $R^2$, $R^3$ et $R^4$ ont la signification mentionnée et X représente un groupe séparable, ou

b) On fait réagir un composé de formule générale

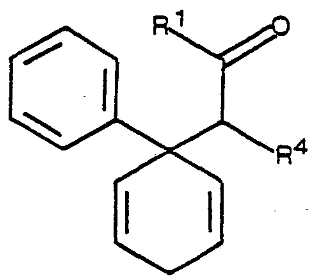

(III)

où $R^1$ et $R^4$ ont la signification mentionnée, dans des conditions réductrices avec une amine de formule générale

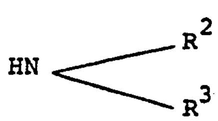

(IV)

où $R^2$ et $R^3$ ont la signification mentionnée, où

c) On fait réagir un composé de formule générale

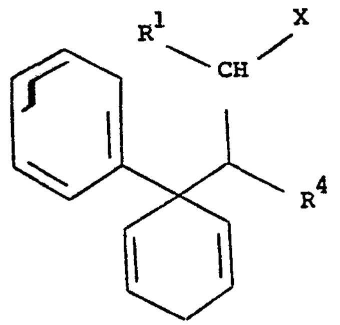

(V)

où $R^1$ et $R^4$ ont la signification mentionnée et où X représente un groupe séparable, avec une amine de formule IV, ou

d) On alcoyle de façon correspondante un composé de formule générale

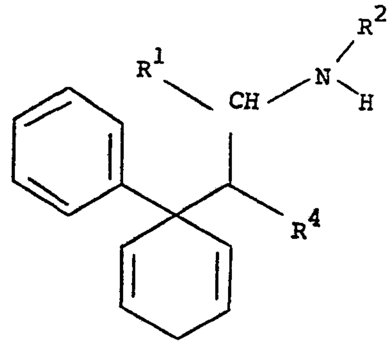

(VI)

où $R^1$, $R^2$ et $R^4$ ont la signification mentionnée, ou

e) On réduit un composé de formule générale

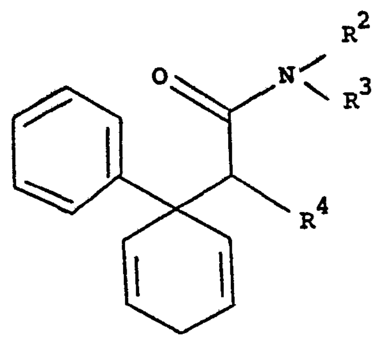

(VII)

où $R^2$, $R^3$ et $R^4$ ont la signification mentionnée, puis, si on le désire, on dédouble le racémate obtenu en ses antipodes optiquement actifs et on transforme éventuellement un produit obtenu en un sel d'addition d'acide.

2. Procédé de préparation de la N,N-diméthyl-1-phényl-2,5-cyclohexadièn-1-éthylamine et de ses sels d'addition d'acides pharmaceutiquement acceptables selon la revendication 1, caractérisé en ce que l'on utilise des composés de départ conformément substitués et, si on le désire, on transforme la base libre obtenue en un sel d'addition d'acide pharmaceutiquement acceptable.